# EUROPEAN PATENT SPECIFICATION

(11) **EP 4 225 198 B1**
(45) Date of publication and mention of the grant of the patent: **27.11.2024**
(21) Application number: 21815298.1
(22) Date of filing: 12.10.2021
(51) Int. Cl.: A61B 46/00, A61B 46/10, A61B 17/34, A61B 46/23

(54) **PROCEDURAL BARRIERS WITH INTEGRATED MEDICAL DEVICE-CONNECTING FEATURES AND METHODS THEREOF**
VERFAHRENSSPERREN MIT INTEGRIERTEN VERBINDUNGSMERKMALEN FÜR MEDIZINISCHE VORRICHTUNGEN UND VERFAHREN DAFÜR
BARRIÈRES PROCÉDURALES À CARACTÉRISTIQUES DE CONNEXION DE DISPOSITIF MÉDICAL INTÉGRÉES ET LEURS PROCÉDÉS

(30) Priority: 13.10.2020 US 202063091142 P
(43) Date of publication of application: 16.08.2023
(73) Proprietor: Bard Access Systems, Inc., Salt Lake City, UT 84116 (US)
(72) Inventor: MISENER, Anthony, K., Bountiful, UT 84010 (US); SOWARDS, Steffan, Salt Lake City, UT 84124 (US)
(74) Representative: Hoffmann Eitle
(86) International application number: PCT/US2021/054593
(87) International publication number: WO 2022/081583

(56) References cited:
- EP-A1- 3 270 817
- EP-A1- 3 673 801
- EP-B1- 3 270 817
- US-A1- 2013 247 921
- US-A1- 2015 148 615
- US-A1- 2015 223 897

## Description

### PRIORITY

This application claims the benefit of priority to U.S. Provisional Application No. 63/091,142, filed October 13, 2020.

### BACKGROUND

Procedural fields are typically established about patients with one or more procedural barriers before medical procedures. For example, sterile fields can be established over or around patients by covering the patients with sterile drapes. Oftentimes, the medical procedures require multi-use medical devices that cannot be sterilized, which mandate placement of such medical devices under the sterile drapes; however, sterile single-use medical devices often need to be functionally connected to the multi-use medical devices. Some existing solutions rely on breaching sterile drapes to make functional connections between sterile single-use medical devices and multi-use medical devices. But breaching such sterile drapes risks contaminating the sterile fields carefully established about the patients. That, and establishing some functional connections such as optical connections between the sterile single-use medical devices and the multi-use medical devices can be difficult, particularly through breaches in the sterile drapes. What is needed are procedural barriers with integrated medical device-connecting features that facilitate establishing safe, functional connections between sterile single-use medical devices and multi-use medical devices.

US 2013/0247921 A1 discloses methods and materials related to portals that can be used to create a channel through a surgical drape without compromising the sterile operating field. For example, a portal can include an applicator frame and sheath that is capable of creating a channel into a sterile operating filed so that a device, whether sterile or not can be used at the time of surgery without compromising the sterility of the surgical field.

US 2015/0223897 A1 discloses a sterile handle for controlling a robotic surgical system from a sterile field.

EP 3 673 801 A1 discloses systems and methods for breaching a sterile field for intravascular placement of a catheter.

WO 2016/146993 A1 discloses a supply line for a robotic arm instrument.

US 2015/0148615 A1 discloses ophthalmic surgical systems, methods and devices.

Disclosed herein are procedural barriers with integrated medical device-connecting features and methods thereof that address the foregoing.

### SUMMARY

The invention is defined by independent claims 1 and 8. Further embodiments are defined by the dependent claims. No methods for treatment of the human or animal body by surgery or therapy are claimed. Disclosed herein is a procedural barrier for a medical procedure. The procedural barrier includes, a sheet of one or more nonwoven materials and an integrated connecting means in the sheet for connecting one or more single-use medical devices in a procedural field to another medical device on a patient-facing side of the sheet. The sheet has a length and a width sufficient to establish the procedural field for the medical procedure on a side of the sheet opposite the patient-facing side of the sheet. The integrated connection means includes an integrated conduit passing through the sheet. The integrated conduit passes through the sheet for establishing an alternative electrical connection, an optical connection, or both the alternative electrical connection and the optical connection across the procedural barrier between a same or different single-use medical device in the procedural field to the other medical device on the patient-facing side of the sheet without compromising the procedural field.

The procedural barrier includes an integrated electrical connector integrated into the sheet for establishing an electrical connection across the procedural barrier by way of the integrated electrical connector.

In some embodiments, the procedural barrier includes the integrated conduit passing through the sheet adjacent to the integrated electrical connector for establishing the optical connection across the procedural barrier by way of the integrated conduit and the electrical connection across the procedural barrier by way of the integrated electrical connector.

In some embodiments, the procedural barrier further includes a fenestration in the sheet adjacent to the integrated electrical connector. The fenestration is covered by a transparent fenestration cover of a polymeric material having sufficient optical transmissibility for establishing the optical connection across the procedural barrier by way of the fenestration cover without compromising the procedural field.

In some embodiments, the polymeric material is a polyethylene, a polypropylene, or a polyurethane.

In some embodiments, the procedural barrier includes the integrated conduit passing through the sheet for establishing both the alternative electrical connection and the optical connection across the procedural barrier by way of the integrated conduit.

In some embodiments, the sheet includes one or more plies of the one-or-more nonwoven materials. Each ply of the one-or-more plies is formed of a nonwoven material selected from a polypropylene and a wood pulp.

In some embodiments, the sheet includes a single ply of spunbond polypropylene.

In some embodiments, the sheet includes a single ply of spunlace wood pulp.

In some embodiments, the sheet includes one ply of meltblown polypropylene between two plies of spunbond polypropylene.

In some embodiments, the procedural barrier is a sterile procedural drape.

Also disclosed is a method of a procedural barrier for a medical procedure. The method includes, a procedural barrier-placing step and a device-connecting step. The barrier-placing step includes placing the procedural barrier over a patient. The barrier-placing step establishes a procedural field for the medical procedure on a side of the procedural barrier opposite a patient-facing side of the procedural barrier. The device-connecting step includes connecting one or more single-use medical devices in the procedural field to another medical device on the patient-facing side of the procedural barrier. The

procedural barrier includes an integrated connecting means in a sheet of one or more nonwoven materials for the connecting of the one-or-more single-use medical devices to the other medical device including an integrated electrical connector and an integrated conduit passing through the sheet. The integrated conduit passes through the sheet for establishing an alternative electrical connection, an optical connection, or both the alternative electrical connection and the optical connection across the procedural barrier between a same or different single-use medical device in the procedural field to the other medical device on the patient-facing side of the sheet without compromising the procedural field.

In some embodiments, the device-connecting step includes establishing the electrical connection across the procedural barrier by way of the integrated electrical connector into the sheet.

In some embodiments, the device-connecting step includes establishing the optical connection across the procedural barrier by way of the integrated conduit passing through the sheet adjacent to the integrated electrical connector.

In some embodiments, the device-connecting step includes establishing the optical connection across the procedural barrier by way of a transparent fenestration cover covering a fenestration in the sheet adjacent to the integrated electrical connector. The fenestration cover is of a polymeric material having sufficient optical transmissibility for establishing the optical connection

In some embodiments, the polymeric material is a polyethylene, polypropylene, or polyurethane.

In some embodiments, the device-connecting step includes establishing both the alternative electrical connection and the optical connection across the procedural barrier by way of the integrated conduit passing through the sheet.

In some embodiments, the sheet includes one or more plies of the one-or-more nonwoven materials, each ply of the one-or-more plies formed of a nonwoven material selected from a polypropylene and a wood pulp.

In some embodiments, the sheet includes a single ply of spunbond polypropylene, a single ply of spunlace wood pulp, or one ply of meltblown polypropylene between two plies of spunbond polypropylene.

In some embodiments, the procedural barrier is a sterile procedural drape.

These and other features of the concepts provided herein will become more apparent to those of skill in the art in view of the accompanying drawings and following description, which describe particular embodiments of such concepts in greater detail.

### DRAWINGS

FIG. 1 illustrates a procedural barrier including an integrated electrical connector and a covered fenestration in accordance with some embodiments.
FIG. 2 illustrates a procedural barrier including an integrated electrical connector and integrated conduit in accordance with the invention.
FIG. 3 illustrates a procedural barrier including an integrated conduit in accordance with some embodiments.
FIG. 4 illustrates a procedural barrier including a covered fenestration in accordance with some embodiments.
FIG. 5 illustrates a functional connection from a PICC in a sterile field to a relay module of a console outside the sterile field in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

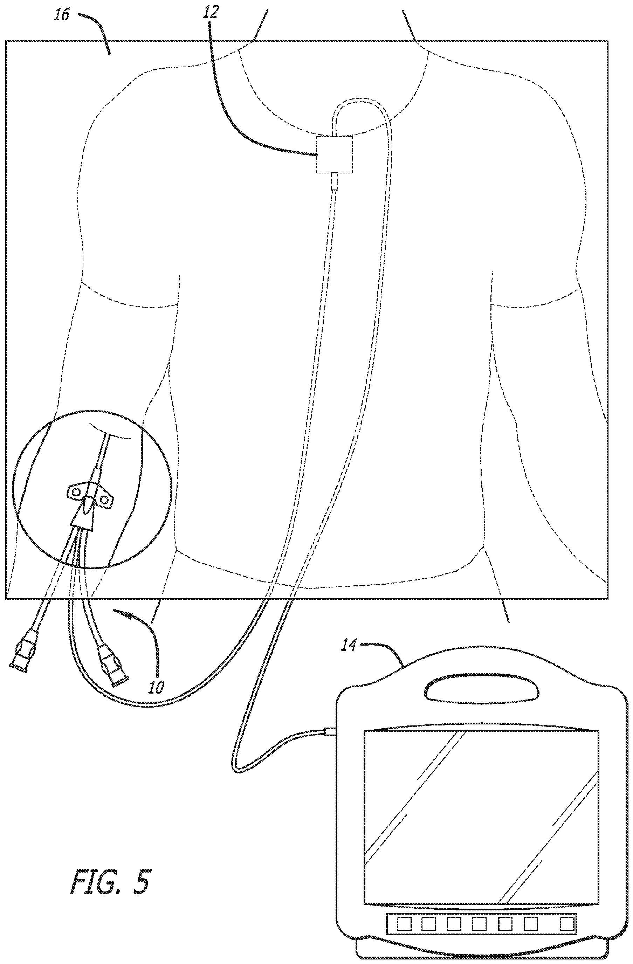

As set forth above, medical procedures often require multi-use medical devices that cannot be sterilized, which mandate placement of such medical devices under sterile drapes where it is difficult to functionally connect them to sterile single-use medical devices in sterile fields over or around patients established by the sterile drapes. As shown in FIG. 5, some existing solutions rely on breaching sterile drapes to make functional connections between sterile single-use medical devices and multi-use medical devices. Indeed, FIG. 5 illustrates a functional connection (e.g. a power-and-data connection) from a peripherally inserted central catheter ("PICC") 10 in a sterile field to a relay module 12 of a console 14 outside the sterile field by way of a breach in a sterile drape 16. But breaching such sterile drapes risks contaminating the sterile fields carefully established about the patients. That, and establishing some functional connections such as optical connections between the sterile single-use medical devices and the multi-use medical devices can be difficult, particularly through breaches in the sterile drapes.

Disclosed herein are procedural barriers with integrated medical device-connecting features and methods thereof that facilitate establishing safe, functional connections between sterile single-use medical devices and other medical devices such as multi-use medical devices.

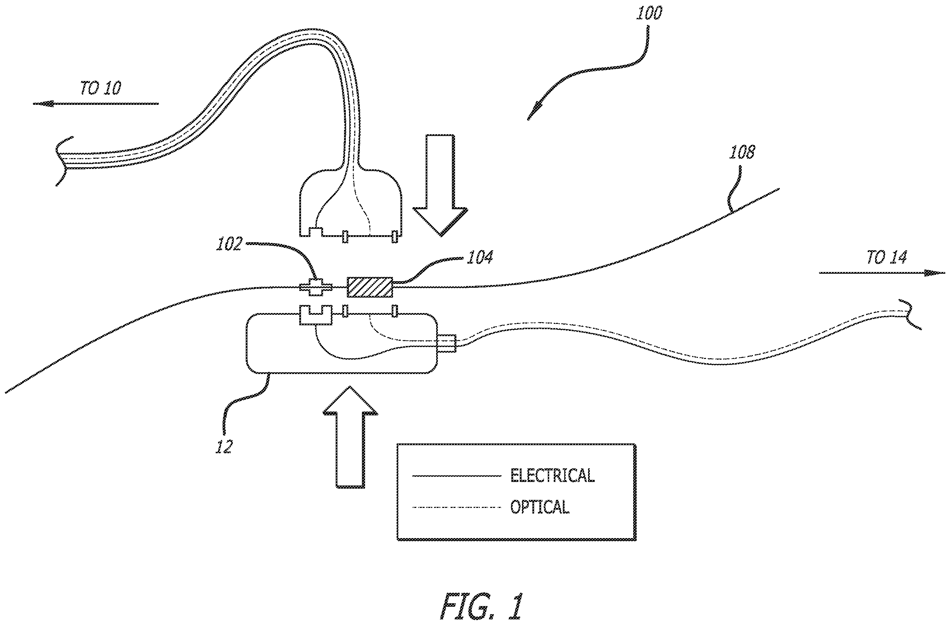

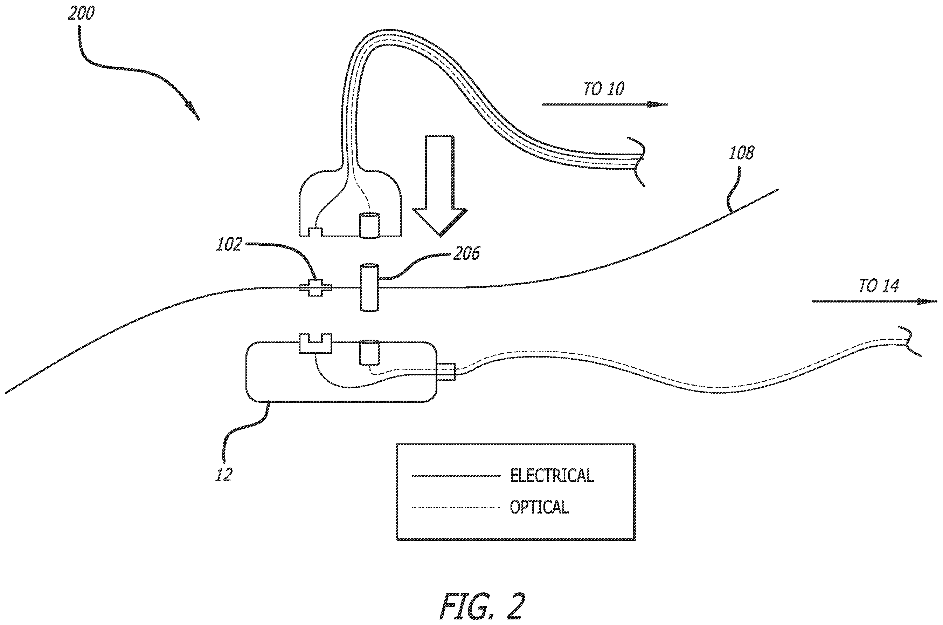

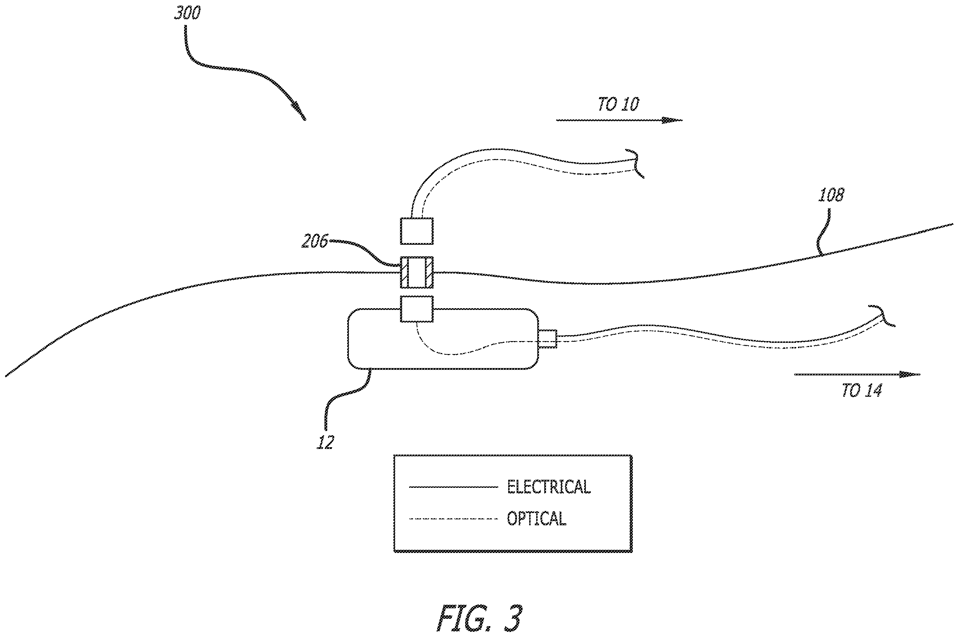

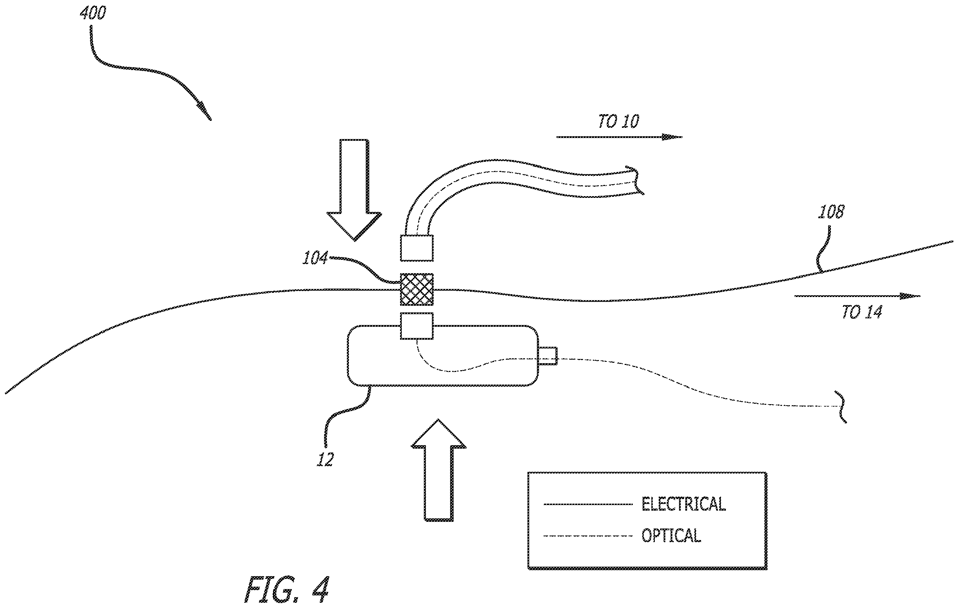

FIG. 1 illustrates a procedural barrier 100 including an integrated electrical 102 connector and a fenestration covered by a fenestration cover 104 in accordance with some embodiments. FIG. 2 illustrates a procedural barrier 200 including the integrated electrical connector 102 and integrated conduit 206 in accordance with the invention. FIG. 3 illustrates a procedural barrier 300 including the integrated conduit 260 in accordance with some embodiments. FIG. 4 illustrates a procedural barrier 400 including the fenestration cover 104 in accordance with some embodiments.

Each procedural barrier of the procedural barriers 100, 200, 300, and 400 disclosed herein is for a medical procedure but is not limited thereto. As set forth below, each procedural barrier of the procedural barriers 100, 200, 300, and 400 includes a sheet 108 of one or more nonwoven materials and an integrated connecting means in the sheet 108 for functionally connecting one or more single-use medical devices (e.g. the PICC 10 having an electrocardiogram ["ECG"] stylet, an optical-fiber stylet, or both) in a procedural field over or around a patient to another medical device such as a multi-use medical device (e.g., the console 14 by way of the relay module 12) on a patient-facing side of the procedural barrier 100, 200, 300, or 400 or the sheet 108 thereof (i.e., under the procedural barrier 100, 200, 300, or 400 or sheet 108 thereof). In some embodiments, the procedural barrier 100, 200, 300, or 400 is a sterile drape. In such embodiments, the procedural field is a sterile field.

The sheet 108 has a length and a width sufficient to establish the procedural field for the medical procedure on a side of the sheet 108 opposite the patient-facing side of the sheet 108. Again, such a procedural field can be a sterile field when the procedural barrier 100, 200, 300, or 400 is a sterile drape.

The sheet 108 includes a single ply of the one-or-more nonwoven materials or two or more plies of the one-or-more nonwoven materials. The nonwoven material of any ply of the sheet 108 can be a polypropylene or a wood pulp. In other words, the single ply or each ply of the two-or-more plies of the sheet 108 can independently be a polypropylene or a wood pulp. For example, the sheet 108 can be a single ply of spunbond polypropylene. Alternatively, the sheet 108 can be a single ply of spunlace wood pulp. In another example, the sheet 108 includes one ply of meltblown polypropylene between two plies of spunbond polypropylene.

The integrated connection means is selected from at least an integrated electrical connector 102 integrated into the sheet 108, an integrated conduit 206 passing through the sheet 108, and both the integrated electrical connector 102 and the integrated conduit 206.

As shown in FIGS. 1 and 2, the integrated electrical connector 102 is integrated into the sheet 108 for establishing an electrical connection across the procedural barrier 100 or 200 between a single-use medical device (e.g., the PICC 10 with the ECG stylet) in the procedural field to the multi-use medical device (e.g., the console 14 by way of the relay module 12) on the patient-facing side of the procedural barrier 100 or 200 or the sheet 108 thereof. Notably, the integrated electrical connector 102 enables functional connections such as the foregoing electrical connection without compromising the procedural field. To effectuate the electrical connection, the integrated electrical connector 102 can be a molded piece snapped together over a fenestration in the sheet 108, wherein the molded piece has electrical contacts complementary to both the single-use medical device and the multi-use medical device.

As shown in FIGS. 2 and 3, the integrated conduit 206 passes through the sheet 108 for establishing an alternative electrical connection to that of the integrated electrical connector 102, an optical connection, or both the alternative electrical connection and the optical connection (e.g., a multimodal connection) across the procedural barrier 200 or 300 between a single-use medical device in the procedural field to the multi-use medical device (e.g., the console 14 by way of the relay module 12) on the patient-facing side of the procedural barrier 200 or 300 or the sheet 108 thereof. When both the integrated electrical connector 102 and the integrated conduit 206 are present in the same sheet 108 as shown in FIG. 2 for the procedural barrier 200, the integrated conduit 206 passes through the sheet 108 adjacent to the integrated electrical connector 102 for establishing, for example, an electrical connection across the procedural barrier 200 by way of the integrated electrical connector 102 and an optical connection across the procedural barrier 200 by way of the integrated conduit 206. Notably, the integrated conduit 206 enables functional connections such as the foregoing alternative electrical and optical connections without compromising the procedural field. To effectuate the functional connections, the integrated conduit 206 can be a molded or extruded piece inserted into a fenestration in the sheet 108, wherein the molded or extruded piece has electrical contacts therein complementary to both the single-use medical device and the multi-use medical device, an intermediate optical fiber-containing ferrule therein complementary to both the single-use medical device and the multi-use medical device, or both. Being molded or extruded, the integrated conduit can have alignment feature configured to align the functional connections.

As shown in FIGS. 1 and 4, the procedural barrier 100 and 400 can include a fenestration in the sheet 108 covered by the transparent fenestration cover 104 for establishing an optical connection across the procedural barrier 100 or 400 between a single-use medical device in the procedural field to the multi-use medical device (e.g., the console 14 by way of the relay module 12) on the patient-facing side of the procedural barrier 100 or 400 or the sheet 108 thereof. When both the integrated electrical connector 102 and the fenestration cover 104 are present in the same sheet 108 as shown in FIG. 1 for the procedural barrier 100, the fenestration cover 104 covers a fenestration in the sheet 108 adjacent to the integrated electrical connector 102 for establishing, for example, an electrical connection across the procedural barrier 100 by way of the integrated electrical connector 102 and an optical connection across the procedural barrier 100 by way of the fenestration cover 104. Notably, the fenestration cover 104 enables functional connections such as the foregoing optical connection without compromising the procedural field. To effectuate the optical connection, the fenestration cover 104 can be a transparent polymeric material adhered onto the sheet 108 or between the two-or-more plys of the sheet 108, wherein the fenestration cover 104 has a thickness sufficient for maintaining an integrity of the fenestration cover 104 while establishing the optical connection between, for example, complementarily magnetized optical connectors of the single-use medical device and the multi-use medical device, as well as an optical transmissibility sufficient for transmitting optical signals across the fenestration cover 104.

The fenestration cover 104 includes a single non-laminated transparent sheet of the polymeric material or two-or-more layers of same or different polymeric materials laminated together in a laminated transparent sheet. The polymeric material of the transparent sheet can be a porous, breathable polymeric film such as a polyethylene, a polypropylene, or a polyurethane; however, a porosity of the breathable polymeric film should not be such that optical signals through the fenestration cover 104 are appreciably scattered. For the laminated transparent sheet, each layer of the two-or-more layers can include a same or different polymeric material than an adjacent layer of the two-or-more layers of the laminated transparent sheet.

### Methods

Methods include methods of using a procedural barrier such as the procedural barrier 100, 200, 300, or 400 for a medical procedure. Such a method includes, a procedural barrier-placing step and a device-connecting step.

The barrier-placing step includes placing the procedural barrier 100, 200, 300, or 400 over a patient. The barrier-placing step establishes a procedural field for the medical procedure on a side of the procedural barrier 100, 200, 300, or 400 opposite a patient-facing side of the procedural barrier 100, 200, 300, or 400. As set forth above, the procedural barrier 100, 200, 300, or 400 includes an integrated connecting means in the sheet 108 of the one-or-more nonwoven materials for the connecting of one or more single-use medical devices to another medical device such as a multi-use medical device, wherein the integrated connecting means is selected from the integrated electrical connector 102 integrated into the sheet 108, the integrated conduit 206 passing through the sheet 108, or both the integrated electrical connector 102 and the integrated conduit 206.

The device-connecting step includes connecting the one-or-more single-use medical devices (e.g., the PICC 10) in the procedural field to the multi-use medical device (e.g., the console 14 by way of the relay module 12) on the patient-facing side of the procedural barrier 100, 200, 300, or 400. For example, the device-connecting step includes establishing an electrical connection across the procedural barrier 100 or 200 by way of the integrated electrical connector 102 integrated into the sheet 108 or the integrated conduit 206 passing through the sheet 108. In another example, the device-connecting step includes establishing an optical connection across the procedural barrier 200 or 300 by way of the integrated conduit 206 passing through the sheet 108, optionally together with the electrical connection by way of the integrated conduit 206. In yet another example, the device-connecting step includes establishing the optical connection across the procedural barrier 100 or 400 by way of the transparent fenestration cover 104 covering the fenestration in the sheet 108, optionally along with the electrical connection by way of the integrated electrical connector 102.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A procedural barrier (200) for a medical procedure, comprising:
a sheet (108) of one or more nonwoven materials, the sheet (108) having a length and a width sufficient to establish a procedural field for the medical procedure on a side of the sheet opposite a patient-facing side of the sheet; and
an integrated connecting means in the sheet (108) for connecting one or more single-use medical devices in the procedural field to another medical device on the patient-facing side of the sheet, the integrated connection means comprising:
an integrated electrical connector integrated into the sheet for establishing an electrical connection across the procedural barrier between the single-use medical device in the procedural field to the other medical device on the patient-facing side of the sheet without compromising the procedural field, and
an integrated conduit (206) passing through the sheet (108) for establishing an alternative electrical connection, an optical connection, or both the alternative electrical connection and the optical connection across the procedural barrier (200) between the same or a different single-use medical device in the procedural field to the other medical device on the patient-facing side of the sheet without compromising the procedural field.

2. The procedural barrier of claim 1, wherein the procedural barrier (200) includes the integrated conduit (206) passing through the sheet adjacent to the integrated electrical connector (102) for establishing the optical connection across the procedural barrier (200) by way of the integrated conduit and the electrical connection across the procedural barrier (200) by way of the integrated electrical connector.

3. The procedural barrier of claim 1, further comprising a fenestration in the sheet (108) adjacent to the integrated electrical connector (102), the fenestration covered by a transparent fenestration cover (104) of a polymeric material having sufficient optical transmissibility for establishing the optical connection across the procedural barrier by way of the fenestration cover (104) without compromising the procedural field, optionally
wherein the polymeric material is a polyethylene, a polypropylene, or a polyurethane.

4. The procedural barrier of claim 1, wherein the procedural barrier (200) includes the integrated conduit (206) passing through the sheet for establishing both the alternative electrical connection and the optical connection across the procedural barrier (200) by way of the integrated conduit (206).

5. The procedural barrier of any claim of claims 1-4, wherein the sheet (108) includes one or more plies of the one-or-more nonwoven materials, each ply of the one-or-more plies formed of a nonwoven material selected from a polypropylene and a wood pulp.

6. The procedural barrier of claim 5, wherein the sheet (108) includes a single ply of spunbond polypropylene, or
wherein the sheet (108) includes a single ply of spunlace wood pulp, or
wherein the sheet (108) includes one ply of meltblown polypropylene between two plies of spunbond polypropylene.

7. The procedural barrier of any claim of claims 1-6, wherein the procedural barrier (200) is a sterile procedural drape.

8. A method of using the procedural barrier (200) of any preceding claim for a medical procedure, comprising:
placing the procedural barrier (200) over a patient, thereby establishing a procedural field for the medical procedure on a side of the procedural barrier opposite a patient-facing side of the procedural barrier; and
connecting one or more single-use medical devices in the procedural field to another medical device on the patient-facing side of the procedural barrier.

9. The method of claim 8, wherein connecting the one-or-more single-use medical devices in the procedural field to the other medical device on the patient-facing side of the procedural barrier includes establishing an electrical connection across the procedural barrier by way of the integrated electrical connector (102) into the sheet (108).

10. The method of claim 9, wherein connecting the one-or-more single-use medical devices in the procedural field to the other medical device on the patient-facing side of the procedural barrier includes establishing the optical connection across the procedural barrier by way of the integrated conduit (206) passing through the sheet (108) adjacent to the integrated electrical connector (102).

11. The method of claim 9, wherein connecting the one-or-more single-use medical devices in the procedural field to the other medical device on the patient-facing side of the procedural barrier includes establishing the optical connection across the procedural barrier by way of a transparent fenestration cover (104) covering a fenestration in the sheet adjacent to the integrated electrical connector, the fenestration cover (104) of a polymeric material having sufficient optical transmissibility for establishing the optical connection, optionally
wherein the polymeric material is a polyethylene, polypropylene, or polyurethane.

12. The method of claim 8, wherein connecting the one-or-more single-use medical devices in the procedural field to the other medical device on the patient-facing side of the procedural barrier includes establishing both the alternative electrical connection and the optical connection across the procedural barrier (200) by way of the integrated conduit (206) passing through the sheet.

13. The method of any claim of claims 8-12, wherein the sheet (108) includes one or more plies of the one-or-more nonwoven materials, each ply of the one-or-more plies formed of a nonwoven material selected from a polypropylene and a wood pulp, optionally
wherein the sheet (108) includes a single ply of spunbond polypropylene, a single ply of spunlace wood pulp, or one ply of meltblown polypropylene between two plies of spunbond polypropylene.

14. The method of any claim of claims 8-13, wherein the procedural barrier (200) is a sterile procedural drape.

## Patentansprüche

1. Verfahrenssperre (200) für einen medizinischen Eingriff, umfassend:
einen Bogen (108) aus einem oder mehreren Vliesmaterialien, wobei der Bogen (108) eine Länge und eine Breite aufweist, die ausreichen, um einen Eingriffsbereich für den medizinischen Eingriff auf einer Seite des Bogens gegenüber einer dem Patienten zugewandten Seite des Bogens herzustellen; und
ein integriertes Verbindungsmittel in dem Bogen (108) zum Verbinden einer oder mehrerer medizinischer Vorrichtungen zum einmaligen Gebrauch in dem Eingriffsbereich mit einer anderen medizinischen Vorrichtung auf der dem Patienten zugewandten Seite des Bogens, wobei das integrierte Verbindungsmittel Folgendes umfasst:
einen integrierten elektrischen Verbinder, der in den Bogen integriert ist, um eine elektrische Verbindung über die Verfahrenssperre zwischen der medizinischen Vorrichtung zum einmaligen Gebrauch im Eingriffsbereich mit der anderen medizinischen Vorrichtung auf der dem Patienten zugewandten Seite des Bogens herzustellen, ohne den Eingriffsbereich zu kompromittieren, und eine integrierte Leitung (206), die durch den Bogen (108) verläuft, um eine alternative elektrische Verbindung, eine optische Verbindung oder sowohl die alternative elektrische Verbindung als auch die optische Verbindung über die Verfahrenssperre (200) zwischen der gleichen oder einer unterschiedlichen medizinischen Vorrichtung zum einmaligen Gebrauch im Eingriffsbereich mit der anderen medizinischen Vorrichtung auf der dem Patienten zugewandten Seite des Bogens herzustellen, ohne den Eingriffsbereich zu kompromittieren.

2. Verfahrenssperre nach Anspruch 1, wobei die Verfahrenssperre (200) die integrierte Leitung (206) einschließt, die durch den Bogen angrenzend an den integrierten elektrischen Verbinder (102) verläuft, um die optische Verbindung über die Verfahrenssperre (200) mittels der integrierten Leitung und die elektrische Verbindung über die Verfahrenssperre (200) mittels des integrierten elektrischen Verbinders herzustellen.

3. Verfahrenssperre nach Anspruch 1, weiter umfassend eine Fensterung in dem Bogen (108) angrenzend an den integrierten elektrischen Verbinder (102), wobei die Fensterung durch eine transparente Fensterungsabdeckung (104) aus einem polymeren Material abgedeckt ist, das eine ausreichende optische Durchlässigkeit aufweist, um die optische Verbindung über die Verfahrenssperre mittels der Fensterungsabdeckung (104) herzustellen, ohne den Eingriffsbereich zu kompromittieren, optional
wobei das polymere Material ein Polyethylen, ein Polypropylen oder ein Polyurethan ist.

4. Verfahrenssperre nach Anspruch 1, wobei die Verfahrenssperre (200) die integrierte Leitung (206) einschließt, die durch den Bogen verläuft, um sowohl die alternative elektrische Verbindung als auch die optische Verbindung über die Verfahrenssperre (200) mittels der integrierten Leitung (206) herzustellen.

5. Verfahrenssperre nach einem der Ansprüche 1 - 4, wobei der Bogen (108) eine oder mehrere Lagen der einen oder mehreren Vliesmaterialien einschließt, wobei jede Lage der einen oder mehreren Lagen aus einem Vliesmaterial gebildet ist, das aus einem Polypropylen und einem Holzzellstoff ausgewählt ist.

6. Verfahrenssperre nach Anspruch 5, wobei der Bogen (108) eine einzige Lage aus Spinnvlies-Polypropylen einschließt, oder
wobei der Bogen (108) eine einzige Lage aus Spinngewebe-Holzzellstoff einschließt, oder
wobei der Bogen (108) eine Lage aus schmelzgeblasenem Polypropylen zwischen zwei Lagen aus Spinnvlies-Polypropylen einschließt.

7. Verfahrenssperre nach einem der Ansprüche 1 - 6, wobei die Verfahrenssperre (200) ein steriles Eingriffstuch ist.

8. Verfahren zum Verwenden der Verfahrenssperre (200) nach einem vorstehenden Anspruch für einen medizinischen Eingriff, umfassend:
Platzieren der Verfahrenssperre (200) über einem Patienten, wodurch ein Eingriffsbereich für den medizinischen Eingriff auf einer Seite der Verfahrenssperre gegenüber einer dem Patienten zugewandten Seite der Verfahrenssperre hergestellt wird; und
Verbinden einer oder mehrerer medizinischer Vorrichtungen zum einmaligen Gebrauch in dem Eingriffsbereich mit einer anderen medizinischen Vorrichtung auf der dem Patienten zugewandten Seite der Verfahrenssperre.

9. Verfahren nach Anspruch 8, wobei das Verbinden der einen oder mehreren medizinischen Vorrichtungen zum einmaligen Gebrauch in dem Eingriffsbereich mit der anderen medizinischen Vorrichtung auf der dem Patienten zugewandten Seite der Verfahrenssperre das Herstellen einer elektrischen Verbindung über die Verfahrenssperre mittels des in den Bogen (108) integrierten elektrischen Verbinders (102) einschließt.

10. Verfahren nach Anspruch 9, wobei das Verbinden der einen oder mehreren medizinischen Vorrichtungen zum einmaligen Gebrauch im Eingriffsbereich mit der anderen medizinischen Vorrichtung auf der dem Patienten zugewandten Seite der Verfahrenssperre das Herstellen der optischen Verbindung über die Verfahrenssperre mittels der integrierten Leitung (206) einschließt, die durch den Bogen (108) angrenzend an den integrierten elektrischen Verbinder (102) verläuft.

11. Verfahren nach Anspruch 9, wobei das Verbinden der einen oder mehreren medizinischen Vorrichtungen zum einmaligen Gebrauch in dem Eingriffsbereich mit der anderen medizinischen Vorrichtung auf der dem Patienten zugewandten Seite der Verfahrenssperre das Herstellen der optischen Verbindung über die Verfahrenssperre mittels einer transparenten Fensterungsabdeckung (104) einschließt, die eine Fensterung in dem Bogen angrenzend an den integrierten elektrischen Verbinder abdeckt, wobei die Fensterungsabdeckung (104) aus einem polymeren Material besteht, das eine ausreichende optische Durchlässigkeit für die Herstellung der optischen Verbindung aufweist, optional
wobei das polymere Material ein Polyethylen, Polypropylen oder Polyurethan ist.

12. Verfahren nach Anspruch 8, wobei das Verbinden der einen oder mehreren medizinischen Vorrichtungen zum einmaligen Gebrauch im Eingriffsbereich mit der anderen medizinischen Vorrichtung auf der dem Patienten zugewandten Seite der Verfahrenssperre das Herstellen sowohl der alternativen elektrischen Verbindung als auch der optischen Verbindung über die Verfahrenssperre (200) mittels der integrierten Leitung (206), die durch den Bogen verläuft, einschließt.

13. Verfahren nach einem der Ansprüche 8 - 12, wobei der Bogen (108) eine oder mehrere Lagen des einen oder der mehreren Vliesmaterialien einschließt, wobei jede Lage der einen oder mehreren Lagen aus einem Vliesmaterial gebildet ist, das aus einem Polypropylen und einem Holzzellstoff ausgewählt ist, optional
wobei der Bogen (108) eine einzige Lage aus Spinnvlies-Polypropylen, eine einzige Lage aus Spinngewebe-Holzzellstoff oder eine Lage aus schmelzgeblasenem Polypropylen zwischen zwei Lagen aus Spinnvlies-Polypropylen einschließt.

14. Verfahren nach einem der Ansprüche 8 - 13, wobei die Verfahrenssperre (200) ein steriles Eingriffstuch ist.

## Revendications

1. Barrière (200) pour intervention destinée à une procédure médicale, comprenant :
un drap (108) d'un ou plusieurs matériaux non tissés, le drap (108) présentant une longueur et une largeur suffisantes pour établir un champ d'intervention pour la procédure médicale sur un côté du drap opposé au côté du drap tourné vers le patient ; et
des moyens de connexion intégrés dans le drap (108) pour connecter un ou plusieurs dispositifs médicaux à usage unique dans le champ d'intervention à un autre dispositif médical sur le côté du drap tourné vers le patient, les moyens de connexion intégrés comprenant :
un connecteur électrique intégré qui est intégré dans le drap pour établir une connexion électrique à travers la barrière pour intervention entre le dispositif
médical à usage unique dans le champ d'intervention et l'autre dispositif médical sur le côté du drap tourné vers le patient sans compromettre le champ d'intervention, et un conduit intégré (206) passant à travers le drap (108), pour établir une connexion électrique alternative, une connexion optique, ou à la fois la connexion électrique alternative et la connexion optique à travers la barrière (200) pour intervention entre le même dispositif médical à usage unique ou un dispositif
médical à usage unique différent dans le champ d'intervention et l'autre dispositif médical sur le côté du drap tourné vers le patient sans compromettre le champ d'intervention.

2. Barrière pour intervention selon la revendication 1, dans laquelle la barrière (200) pour intervention inclut le conduit intégré (206) passant à travers le drap de manière adjacente au connecteur électrique intégré (102) pour établir la connexion optique à travers la barrière (200) pour intervention au moyen du conduit intégré et la connexion électrique à travers la barrière (200) pour intervention au moyen du connecteur électrique intégré.

3. Barrière pour intervention selon la revendication 1, comprenant en outre une fenêtre dans le drap (108) de manière adjacente au connecteur électrique intégré (102), la fenêtre étant recouverte par un revêtement (104) transparent de fenêtre d'un matériau polymère présentant une capacité de transmission optique suffisante pour établir la connexion optique à travers la barrière pour intervention au moyen du revêtement (104) de fenêtres sans compromettre le champ d'intervention, éventuellement dans laquelle le matériau polymère est un polyéthylène, un polypropylène ou un polyuréthane.

4. Barrière pour intervention selon la revendication 1, dans laquelle la barrière (200) pour intervention inclut le conduit intégré (206) passant à travers le drap pour établir à la fois la connexion électrique alternative et la connexion optique à travers la barrière (200) pour intervention au moyen du conduit intégré (206).

5. Barrière pour intervention selon l'une quelconque des revendications 1 à 4, dans laquelle le drap (108) inclut une ou plusieurs couches des un ou plusieurs matériaux non tissés, chaque couche des une ou plusieurs couches étant formée d'un matériau non tissé sélectionné parmi un polypropylène et une pâte de bois.

6. Barrière pour intervention selon la revendication 5, dans laquelle le drap (108) inclut une couche simple de polypropylène filé-lié, ou
dans laquelle le drap (108) inclut une couche simple d'une pâte de bois hydroliée, ou dans laquelle le drap (108) inclut une couche de polypropylène de fusion-soufflage entre deux couches de polypropylène filé-lié.

7. Barrière pour intervention selon l'une quelconque des revendications 1 à 6, dans laquelle la barrière (200) pour intervention est un drap stérile pour intervention.

8. Procédé d'utilisation de la barrière (200) pour intervention selon une quelconque revendication précédente
pour une procédure médicale, comprenant :
le fait de positionner la barrière (200) pour intervention sur un patient, permettant ainsi d'établir un champ d'intervention pour la procédure médicale sur un côté de la barrière pour intervention opposé au côté tourné vers le patient de la barrière pour intervention ; et
le fait de connecter un ou plusieurs dispositifs médicaux à usage unique dans le champ d'intervention à un autre dispositif médical sur le côté tourné vers le patient de la barrière pour intervention.

9. Procédé selon la revendication 8, dans lequel la connexion des un ou plusieurs dispositifs médicaux à usage unique dans le champ d'intervention à l'autre dispositif médical sur le côté tourné vers le patient de la barrière pour intervention inclut l'établissement d'une connexion électrique à travers la barrière pour intervention au moyen du connecteur électrique intégré (102) dans le drap (108).

10. Procédé selon la revendication 9, dans lequel la connexion des un plusieurs dispositifs médicaux à usage unique dans le champ d'intervention à l'autre dispositif médical sur le côté tourné vers le patient de la barrière pour intervention inclut l'établissement de la connexion optique à travers la barrière pour intervention au moyen du conduit intégré (206) passant à travers le drap (108) de manière adjacente au connecteur électrique intégré (102).

11. Procédé selon la revendication 9, dans lequel la connexion des un ou plusieurs dispositifs médicaux à usage unique dans le champ d'intervention à l'autre dispositif médical sur le côté tourné vers le patient de la barrière pour intervention inclut l'établissement de la connexion optique à travers la barrière pour intervention au moyen d'un revêtement (104) transparent de fenêtre recouvrant une fenêtre dans le drap de manière adjacente au connecteur électrique intégré, le revêtement (104) de fenêtre d'un matériau polymère présentant une capacité de transmission optique suffisante pour établir la connexion optique, éventuellement
dans lequel le matériau polymère est un polyéthylène, un polypropylène ou un polyuréthane.

12. Procédé selon la revendication 8, dans lequel la connexion des un ou plusieurs dispositifs médicaux à usage unique dans le champ d'intervention à l'autre dispositif médical sur le côté tourné vers le patient de la barrière pour intervention inclut l'établissement à la fois de la connexion électrique alternative et de la connexion optique à travers la barrière (200) pour intervention au moyen du conduit intégré (206) passant à travers le drap.

13. Procédé selon l'une quelconque des revendications 8 à 12, dans lequel le drap (108) inclut une ou plusieurs couches des un ou plusieurs matériaux non tissés, chaque couche des une ou plusieurs couches étant formée d'un matériau non tissé sélectionné parmi un polypropylène et une pâte de bois, éventuellement
dans lequel le drap (108) inclut une couche simple de polypropylène filé-lié, une couche simple d'une pâte de bois hydroliée, ou une couche de polypropylène de fusion-soufflage entre deux couches de polypropylène filé-lié.

14. Procédé selon l'une quelconque des revendications 8 à 13, dans lequel la barrière (200) pour intervention est un drap stérile pour intervention.
